# EUROPÄISCHE PATENTSCHRIFT

(11) **EP 0 370 317 B1**
(45) Veröffentlichungstag und Bekanntmachung des Hinweises auf die Patenterteilung: **02.06.1993**
(21) Anmeldenummer: 89120763.1
(22) Anmeldetag: 09.11.1989
(51) Int. Cl.: C07D 213/61, C07D 213/84, C07D 213/79, C07D 213/81, C07D 215/18

(54) **Verfahren zur Herstellung von substituierten 2-Chlorpyridinen**
Process for the preparation of substituted 2-chloropyridines
Procédé pour la préparation de 2-chloropyridines substitués

(30) Priorität: 22.11.1988 DE 3839332
(43) Veröffentlichungstag der Anmeldung: 30.05.1990
(73) Patentinhaber: BAYER AG, 51368 Leverkusen (DE)
(72) Erfinder: Kaufmann, Dieter, Dr., D-5060 Bergisch Gladbach 2 (DE); Gallenkamp, Bernd, Dr., D-5600 Wuppertal 1 (DE)

(56) Entgegenhaltungen:
- EP-A- 0 032 516
- FR-A- 2 395 262
- US-A- 4 211 873
- CHEMICAL AND PHARMACEUTICAL BULLETIN, Band 36, Nr. 6, 1988, Seiten 2244-2247 ; H. YAMANAKA et al. :"Site-Selectivity in the Reaction of 3-Substituted Pyridine 1-Oxides with Phosphoryl Chloride"
- CHEMISTRY OF THE HETEROCYCLIC N-OXIDES, 1971, Academic Press, London, GB ; A.R. KATRITZKY et al., Seiten 260-265
- PATENT ABSTRACTS OF JAPAN, Band 6, Nr. 57 (C-98)(953), 14. April 1982 & JP-A-56169672
- JOURNAL OF HETEROCYCLIC CHEMISTRY, Band 18, Nr. 5, August 1981, Seiten 939, 940 ; M.L. ASH et al. :"The Synthesis of 2-Chloromethylpyridine from 2-Picoline-N-Oxide"

## Beschreibung

Die vorliegende Erfindung betrifft ein neues Verfahren zur Herstellung von substituierten 2-Chlorpyridinen.

Es ist bekannt, daß man 2-Chlor-5-methyl-pyridin neben 2-Chlor-3-methyl-pyridin, 4-Chlor-3-methyl-pyridin und 3-Chlor-5-methyl-pyridin erhält, wenn man 3-Methyl-pyridin-1-oxid mit Phosphorylchlorid umsetzt (vgl. Weissberger, Chemistry of Heterocyclic Compounds, Pyridine and its Derivatives, Vol. 14, Supplement, Part 2, S. 112, Verlag John Wiley & Sons, New York, 1974). Hauptprodukt dieser Umsetzung ist 4-Chlor-3-methyl-pyridin; der Anteil an 2-Chlor-5-methyl-pyridin liegt im allgemeinen unter 25 %.

Gegenstand einer älteren, im Hinblick auf die vorliegende Anmeldung nicht vorveröffentlichten Patentanmeldung ist die Umsetzung von 3-Methylpyridin-1-oxid mit Phosphorylchlorid in Gegenwart einer basischen organischen Stickstoffverbindung und in Gegenwart eines Verdünnungsmittels (Deutsche Patentanmeldung P 3800179 vom 07.01.88).

Die Chlorierung von 3-substituierten-Pyridinen ist bekannt aus Chem.Pharm.Bull (1988), 36, S.2244-2247 und Chemistry of Heterocyclic N-Oxides,1971, S.260-265. Mit Hilfe der dort beschriebenen Chlorierungsmittel werden jedoch nicht ganz zufriedenstellende Ausbeuten erhalten.

Es wurde nun ein neues Verfahren zur Herstellung von substituierten 2-Chlorpyridin-Derivaten der Formel (I) 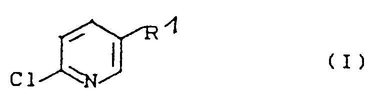
in welcher
- R¹: für Chlor, Alkyl(C₁-C₄), Halogenalkyl(C₁-C₄), Cyanalkyl(C₁-C₄), Alkoxy(C₁-C₄)alkyl(C₁-C₄), Dialkylamino(C₁-C₄)alkyl(C₁-C₄), Carbalkoxy(C₁-C₄) oder 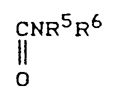 steht,
worin R⁵ und R⁶ gleich oder verschieden sind und für Wasserstoff oder Alkyl (C₁-C₄) stehen
gefunden.

Das neue Verfahren ist dadurch gekennzeichnet, daß man Pyridin-1-oxide der Formel II 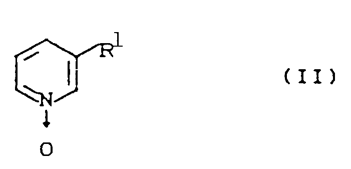
in welcher
R¹ die oben angegebene Bedeutung hat,
mit einem chlorhaltigen Phosphorsäurederivat der Reihe Phosphorsäureesterchloride und Phosphorsäureamidchloride in Gegenwart eines inerten organischen Lösungsmittels und in Gegenwart eines Säureakzeptors bei Temperaturen zwischen -20°C und 200°C umsetzt und das erhaltene Produkt gegebenenfalls weiter auftrennt.

Überraschenderweise gelingt es mit Hilfe des erfindungsgemäßen Verfahrens, auf einfache Weise und mit geringem Aufwand unter Erzielung überraschend hoher Ausbeuten, substituierte Pyridin-1-oxide der Formel (II) durch Umsetzung mit Phosphorsäureesterchloriden bzw. Phosphorsäureamidchloriden in Gegenwart eines Säureakzeptors in die entsprechenden substituierten 2-Chlorpyridine der Formel (I) zu überführen.

Vorteile des erfindungsgemäßen Verfahrens liegen neben der guten Ausbeute am gewünschten Produkt auch darin, daß der Anteil an isomeren Nebenprodukten wesentlich geringer als bei der bisher bekannten Synthesemethode ist. Weiterhin kann man, falls erwünscht, aus dem Reaktionsprodukt die reine Verbindung (I) leicht durch übliche Methoden, z.B. durch destillative Trennung oder durch andere übliche Trennmethoden herstellen. Das erfindungsgemäße Verfahren stellt somit eine wertvolle Bereicherung der Technik dar.

Setzt man 3-Methyl-pyridin-1-oxid mit Phosphorsäurediethylamiddichlorid in Gegenwart der Base Diisopropylamin in Methylenchlorid um, so kann der Reaktionsablauf beim erfindungsgemäßen Verfahren durch das nachstehende Formelschema skizziert werden.

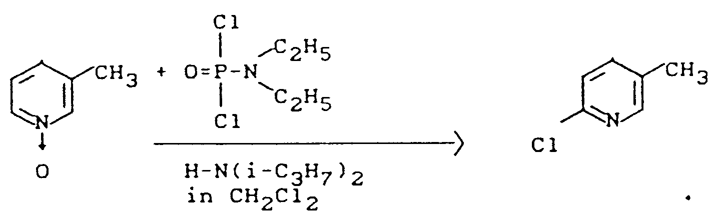

Als nach dem erfindungsgemäßen Verfahren bevorzugt herstellbare Verbindungen der allgemeinen Formel (I) seien genannt:
2-Chlor-5-diisopropylaminomethyl-pyridin
2-Chlor-5-diisobutylaminomethyl-pyridin
2-Chlor-5-methyl-pyridin
2-Chlor-5-ethyl-pyridin
2-Chlor-5-chlormethyl-pyridin
2-Chlor-5-cyanmethyl-pyridin
2-Chlor-5-methoxymethyl-pyridin
2-Chlor-5-ethoxymethyl-pyridin
6-Chlor-nicotinsäuremethylester
6-Chlor-nicotinsäureethylester
6-chlor-nicotinsäuredimethylamid

### Besonders bevorzugt wird folgende Verbindung der Formel (I) hergestellt:

2-Chlor-5-methyl-pyridin.

Die Ausgangsprodukte Pyridin-1-oxide der Formel (II) sowie Phosphorsäureesterchloride bzw. Phosphorsäureamidchloride sind bekannte Verbindungen der organischen Chemie oder lassen sich durch bekannte Verfahren herstellen. Siehe z.B.:
Weissberger, Chemistry of Heterocyclic Compounds, Pyridine and its Derivatives, Vol. 14, Supplement, Part 2, S. 112, Verlag John Wiley & Sons, New York, 1974.

Methoden der Organischen Chemie (Houben-Weyl, Müller), Band 12, 2, S. 212, 239, 383, 445, Georg Thieme Verlag, Stuttgart, 1964.

Die als Ausgangsstoffe für das erfindungsgemäße Verfahren eingesetzten substituierten Pyridin-1-oxide sind durch die Formel (II) allgemein definiert.

In der Formel (II) steht
- R¹: vorzugsweise für Chlor, CH₃, C₂H₅,
CH₂Cl, CH₂CN, CH₂-OCH₃, CH₂-OC₂H₅, CH₂-N(i-C₃H₇)₂, CH₂-N(i-C₄H₉)₂, COOCH₃, COOC₂H₅, CON(CH₃)₂, CON(C₂H₅)₂,
Insbesondere wird 3-Methyl-pyridin-1-oxid als Ausgangsstoff der Formel (II) eingesetzt.

Die beim erfindungsgemäßen Verfahren eingesetzten chlorhaltigen Phosphorsäurederivate der Reihe Phosphorsäureesterchloride und Phosphorsäureamidchloride entstammen vorzugsweise folgenden Verbindungsklassen:
Phosphorsäuremonoalkylesterchloride,
Phosphorsäuredialkylesterchloride,
Phosphorsäurecycloalkylesterchloride,
Phosphorsäurearylesterchloride,
Phosphorsäurediarylesterchloride,
Phosphorsäuremonoalkylamidchloride,
Phosphorsäuredialkylamidchloride,
Phosphorsäurebis(dialkylamid)chloride,
Phosphorsäurecycloalkylamidchloride,
Phosphorsäurearylamidchloride,
Phosphorsäurediarylamidchloride.

Besonders bevorzugt werden 1.Phosphorsäuredialkyl (C₁-C₄)amidchloride und insbesondere Phosphorsäuredimethylamiddichlorid, Phosphorsäurediethylamiddichlorid, Phosphorsäuredipropylamiddichlorid, Phosphorsäurediisopropylamiddichlorid, Phosphorsäuredibutylamiddichlorid und Phosphorsäurediisobutylamiddichlorid eingesetzt und 2. Phosphorsäurealkyl(C₁-C₆)esterchloride und insbesondere Phosphorsäuremethylesterdichlorid, Phosphorsäureethylesterchlorid, Phosphorsäurepropylesterdichlorid, Phosphorsäureisopropylesterdichlorid, Phosphorsäurebutylesterdichlorid, Phosphorsäureisobutylesterdichlorid, Phosphorsäuretert.-butylesterdichlorid, Phosphorsäurepentylesterdichlorid, Phosphorsäurehexylesterdichlorid eingesetzt.

Das erfindungsgemäße Verfahren wird unter Verwendung von inerten organischen Lösungsmitteln durchgeführt. Es kommen dabei praktisch alle inerten organischen Lösungsmittel in Frage. Hierzu gehören vorzugsweise aliphatische oder aromatische Kohlenwasserstoffe wie Pentan, Hexan, Heptan, Octan, Cyclohexan, Methylcyclohexan, Petrolether, Benzin, Ligroin, Benzol, Toluol, Xylol und Tetralin, halogenierte Kohlenwasserstoffe wie Methylenchlorid, Ethylenchlorid, Trichlorethylen, Chloroform, Tetrachlorkohlenstoff, Chlorbenzol und Dichlorbenzol, Ether wie Diethylether, Dipropylether, Diisopropylether, Dibutylether, Methyl-tert-butylether, Glykoldimethylether, Diglykoldimethylether, Tetrahydrofuran und Dioxan, Ketone wie Aceton, Methylethylketon, Diethylketon, Methylisopropylketon, Methyl-isobutylketon und Methyl-tert.-butylketon, Ester wie Essigsäuremethylester, Essigsäureethylester, Essigsäurepropylester, Essigsäurebutylester, Essigsäureamylester, Phthalsäuredimethylester und Phthalsäurediethylester, Nitrile wie Acetonitril und Propionitril, Amide wie Dimethylformamid, Dimethylacetamid und N-Methylpyrrolidon sowie Dimethylsulfoxid und Tetramethylensulfon.

Methylenchlorid, Chloroform und Chlorbenzole werden als organische Lösungsmittel besonders bevorzugt.

Die erfindungsgemäße Umsetzung wird in Gegenwart eines Säureakzeptors durchgeführt. Als Säureakzeptor kommt vorzugsweise eine basische Stickstoffverbindung in Frage. Bevorzugte basische Stickstoffverbindungen sind Dialkylamine wie z.B. Diethylamin, Dipropylamin, Dibutylamin, Diisobutylamin und Di-sec-butylamin, Trialkylamine wie z.B. Triethylamin, Tripropylamin und Tributylamin, Dialkylcycloalkylamine wie z.B. Dimethyl-cyclopentylamin, Diethyl-cyclopentylamin, Dimethyl-cyclohexylamin und Diethylcyclohexylamin, Dialkylaralkylamine wie z.B. Dimethylbenzylamin und Diethylbenzylamin sowie Dialkylarylamine wie z.B. Dimethylanilin sowie Pyridin-derivate wie 2,6-Dimethylpyridin und 2,4,6-Trimethylpyridin.

Diisopropylamin wird als basische organische Stickstoffverbindung besonders bevorzugt.

Besonders erwähnt sei die Herstellung von 2-Chlor-5-methyl-pyridin durch Umsetzung von 3-Methyl-pyridin-1-oxid mit Phosphorsäuredialkylamiddichlorid oder Phosphorsäurealkylesterdichlorid unter Zugabe von Diisopropylamin als Säureakzeptor in Methylenchlorid oder Chlorbenzol.

Beim erfindungsgemäßen Verfahren arbeitet man in einem Temperaturbereich Zwischen -20°C und 200°C, vorzugsweise bei Temperaturen zwischen 0°C und 150°C. Das erfindungsgemäße Verfahren wird im allgemeinen unter Normaldruck durchgeführt. Es ist jedoch auch möglich, unter erhöhtem oder vermindertem Druck zwischen 0,1 und 10 bar zu arbeiten. Zur Durchführung des erfindungsgemäßen Verfahrens setzt man auf 1 Mol substituiertes Pyridin-1-oxid der Formel (II) im allgemeinen zwischen 1 und 10 Mol, vorzugsweise zwischen 1 und 2 Mol, Phosphorsäureesterchlorid oder Phosphorsäureamidchlorid und ebenfalls zwischen 1 und 10 Mol, vorzugsweise zwischen 1 und 2 Mol, der basischen organischen Stickstoffverbindung ein. Der Einsatz von angenähert je 1,5 Mol Phosphorsäureesterchlorid oder Phosphorsäureamidchlorid und Stickstoffverbindung je Mol substituiertes Pyridin-N-oxid wird besonders bevorzugt.

Zur bevorzugten Durchführung des erfindungsgemäßen Verfahrens tropft man ein Gemisch aus einem Phosphorsäureesterchlorid oder Phosphorsäureamidchlorid und einem Amin zu einer Lösung des substituierten Pyridinoxids der Formel (II), und das komplette Reaktionsgemisch wird mehrere Stunden bei der jeweils erforderlichen Temperatur (vorzugsweise im Bereich von 0°C und 150°C) gerührt.
Die Aufarbeitung kann auf übliche Weise durchgeführt werden. Vorzugsweise wird das Reaktionsgemisch nach Filtration a) destilliert, b) extrahiert oder c) mit Wasser versetzt, das organische Lösungsmittel - beispielsweise durch Destillation - entfernt, die wäßrige Phase mit einer wäßrigen Alkalimetall- oder Erdalkalimetallhydroxidlösung wie z.B. Natronlauge auf pH 6 eingestellt und das Reaktionsprodukt daraus weitgehend durch Wasserdampfdestillation entfernt. Der organische Anteil des Wasserdampfdestillats enthält im wesentlichen das Produkt der Formel (I).

Die Reindarstellung der Verbindung der Formel (I) aus dem organischen Anteil des Wasserdampfdestillats kann nach üblichen Methoden, beispielsweise durch eine Feindestillation an einer Füllkörperkolonne erfolgen. Die Gesamtausbeute bei der Herstellung von 2-Chlor-5-methyl-pyridin beträgt ausgehend vom 3-Methyl-pyridin-1-oxid 70 bis 83 % der Theorie.
Das nach dem erfindungsgemäßen Verfahren herstellbare 2-Chlor-5-methylpyridin ist als Zwischenprodukt für Pharmazeutika bekannt (vgl. DE-A 28 12 585).
Weiterhin kann 2-Chlor-5-methyl-pyridin als Zwischenprodukt für die Herstellung von insektiziden Nitromethylen-Derivaten eingesetzt werden (vgl. EP-A 163 855).

### Herstellungsbeispiel 1

### 2-Chlor-5-methylpyridin

Zu einer Lösung von 27,3 g (0,25 Mol) 3-Methylpyridin-1-oxid in 300 ml Methylenchlorid wird eine Lösung von 71,3 g (0,375 Mol) Phosphorsäurediethylamiddichlorid und 37,9 g (0,375 Mol) Diisopropylamin in 100 ml Methylenchlorid getropft. Nach 5 Stunden bei Raumtemperatur wird der Ansatz abgesaugt, der Filterkuchen einmal mit 50 ml Methylenchlorid gewaschen und das Lösungsmittel im Vakuum abgezogen. Der Rückstand wird mit 100 ml Wasser versetzt, mit 20 %iger Natronlauge auf pH 6 eingestellt und einer Wasserdampfdestillation unterworfen. Während der Destillation wird der Inhalt des Destillierkolbens durch kontinuierliche Zugabe von 20 %iger Natronlauge ständig auf pH 6 gehalten. Das Destillat wird dreimal mit je 100 ml Methylenchlorid extrahiert. Die vereinigten Extrakte werden am Rotationsverdampfer konzentriert und man erhält 26,6 g (83 % der Theorie) eines Gemisches aus 82 % 2-Chlor-5-methylpyridin und 18 % 2-Chlor-3-methylpyridin. Die Struktur wird durch ¹H-NMR-Spektren gesichert.

¹H-NMR (CDCl₃, 200 MHz): δ = 8,16 (6-H, ddq) 7,43 (4-H, ddq), 7,16 (3-H, br.d.), 2,26 ppm (CH₃, br.s).

Das reine 2-Chlor-5-methylpyridin kann durch fraktionierte Destillation abgetrennt werden.

### Herstellungsbeispiel 2

### 2-Chlor-5-methylpyridin

Zu einer Lösung von 27,3 g (0,25 Mol) 3-Methylpyridin-1-oxid in 400 ml Chlorbenzol wird unter Stickstoff eine Lösung von 92,2 g (0,375 Mol) Phosphorsäurediisobutylamiddichlorid und 37,9 g (0,375 Mol) Diisopropylamin in 150 ml Chlorbenzol so zugetropft, daß die Innentemperatur nicht über 35°C ansteigt. Nach vollständiger Zugabe wird noch 15 Stunden bei Raumtemperatur nachgerührt, 4 Stunden unter Rückfluß erhitzt, dann der Ansatz abgesaugt, der Filterkuchen einmal mit 50 ml Chlorbenzol gewaschen und das Lösungsmittel im Vakuum (50 mbar) über eine 40 cm Füllkörperkolonne abdestilliert. Der Rückstand wird mit 100 ml Wasser versetzt, mit 20%iger Natronlauge auf pH 6 eingestellt und einer Wasserdampfdestillation unterworfen. Während der Destillation wird der Inhalt des Destillierkolbens durch kontinuierliche Zugabe von 20%iger Natronlauge ständig auf pH 6 gehalten (Kontrolle mit pH-Elektrode). Das Destillat wird dreimal mit je 100 ml Methyl-tert-butylether extrahiert. Die vereinigten Extrakte werden am Rotationsverdampfer konzentriert und man erhält 23,8 g (75 %) eines Produktgemisches, das nach dem Gaschromatogramm folgende Zusammensetzung aufweist:
5 Gew.-% Chlorbenzol
2 Gew.-% Diisobutylamin
11 Gew.-% 2-Chlor-3-methylpyridin
82 Gew.-% 2-Chlor-5-methylpyridin.

### Herstellungsbeispiel 3

### 2-Chlor-5-methylpyridin

Zu einer Lösung von 115.0 g (0.75 mol) Phosphorylchlorid in 200 ml Chlorbenzol bei Raumtemperatur unter Stickstoff werden 50.6 g (0.5 mol) Dipropylamin in 40 min so getropft, daß die Temperatur nicht über 60°C steigt. Es wird 24 Stunden unter Rückfluß erhitzt und anschließend 25 g abdestilliert (Kopftemperatur 122 - 128°C). Die Reaktionslösung und 50.6 g (0.5 mol) Diisopropylamin werden innerhalb von 100 min gleichzeitig zu einer 60°C warmen Lösung von 36.0 g (0.33 mol) β-Picolin-N-oxid in 130 ml Chlorbenzol getropft. Es wird 2 Stunden auf 60°C erwärmt, das Diisopropylaminhydrochlorid abgesaugt und mit 50 ml Chlorbenzol gewaschen. Das Filtrat wird 1 Stunde mit 200 ml halbkonz. Salzsäure verrührt, die wäßrige Phase dann mit konz. Natronlauge auf pH 6 gestellt und zweimal mit je 100 ml Toluol extrahiert. Nach Abdestillieren des Lösungsmittels erhält man 31.1 g eines Gemisches aus 81 % 2-Chlor-5-methylpyridin und 19 % 2-Chlor-3-methylpyridin.

### Herstellungsbeispiel 4

### 2-Chlor-5-methylpyridin

Zu einer Lösung von 27.3 g (0.25 mol) β-Picolin-N-oxid in 250 ml Chlorbenzol werden unter Stickstoff bei 25°C unter Kühlung parallel 81.5 g (0.5 mol) Phosphorsäureethylesterdichlorid und 50.6 g (0.5 mol) Diisopropylamin in 45 min getropft. Es wird anschließend 3 Stunden bei Raumtemperatur nachgerührt, abgesaugt, der Filterkuchen mit 50 ml Chlorbenzol gewaschen und die Chlorbenzollösung fraktioniert destilliert. Man erhält 22.2 g (70 %) eines Gemisches aus 81 % 2-Chlor-5-methylpyridin und 19 % 2-Chlor-3-methylpyridin.

## Patentansprüche

1. Verfahren zur Herstellung von substituierten 2-Chlorpyridin-Derivaten der Formel (I) 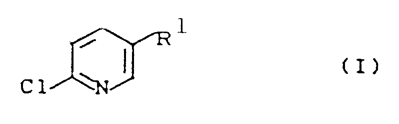 in welcher
R ¹ für Chlor, Alkyl(C₁-C₄),
Halogenalkyl(C₁-C₄), Cyanalkyl(C₁-C₄),
Alkoxy(C₁-C₄)alkyl(C₁-C₄), Dialkylamino(C₁-C₄)alkyl(C₁-C₄), Carbalkoxy-(C₁-C₄) oder 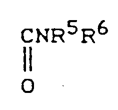 steht,
worin R⁵ und R⁶ gleich oder verschieden sind und für Wasserstoff oder Alkyl (C₁-C₄) stehen
dadurch gekennzeichnet, daß man Pyridin-1-oxide der Formel (II) 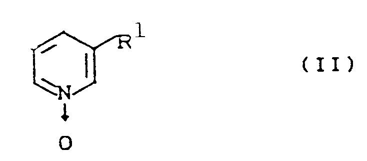 in welcher
R¹ die oben angegebene Bedeutung hat,
mit einem chlorhaltigen Phosphorsäurederivat der Reihe Phosphorsäureesterchloride und Phosphorsäureamidchloride in Gegenwart eines inerten organischen Lösungsmittels und in Gegenwart eines Säureakzeptors bei Temperaturen zwischen -20°C und 200°C umsetzt und das erhaltene Produkt gegebenenfalls weiter auftrennt.

2. Verfahren gemäß Anspruch 1, dadurch gekennzeichnet, daß bei der Umsetzung als chlorhaltiges Phosphorsäurederivat
Phosphorsäuremonoalkylesterchlorid,
Phosphorsäuredialkylesterchlorid,
Phosphorsäurecycloalkylesterchlorid,
Phosphorsäurearylesterchlorid,
Phosphorsäurediarylesterchlorid,
Phosphorsäuremonoalkylamidchlorid,
Phosphorsäuredialkylamidchlorid,
Phosphorsäurebis(dialkylamid)chlorid,
Phosphorsäurecycloalkylamidchlorid,
Phosphorsäurearylamidchlorid,
Phosphorsäurediarylamidchlorid
eingesetzt wird.

3. Verfahren nach Anspruch 1 und 2, dadurch gekennzeichnet, daß man als Säureakzeptoren eine basische organische Stickstoffverbindung der Reihe Dialkylamine, Trialkylamine, Dialkyl-cycloalkylamine, Dialkylaralkylamine, Dialkylarylamine und Pyridinderivate einsetzt.

4. Verfahren gemäß Anspruch 1 bis 3, dadurch gekennzeichnet, daß als Säureakzeptor Diisopropylamin eingesetzt wird.

5. Verfahren gemäß Anspruch 1 bis 4, dadurch gekennzeichnet, daß als inerte organische Lösungsmittel Methylenchlorid, Chloroform oder Chlorbenzole eingesetzt werden.

6. Verfahren gemäß Anspruch 1 bis 5, dadurch gekennzeichnet, daß die Umsetzung bei Temperaturen zwischen 0°C und 150°C durchgeführt wird.

7. Verfahren gemäß Anspruch 1 bis 6, dadurch gekennzeichnet, daß man zu einer Lösung von 1 Mol des substituierten Pyridin-N-oxids der Formel (III) in einem Verdünnungsmittel eine äquimolare Mischung von jeweils 1 bis 10 Mol eines Phosphorsäureesterchlorids oder Phosphorsäureamidchlorids und eines Amins tropft und das Reaktionsgemisch mehrere Stunden gerührt wird.

8. Verfahren gemäß Anspruch 1 bis 7, dadurch gekennzeichnet, daß man das bei der Umsetzung entstehende Reaktionsgemisch destilliert, extrahiert oder einer Wasserdampfdestillation unterwirft.

9. Verfahren gemäß Anspruch 1 bis 8, dadurch gekennzeichnet, daß man zur Herstellung von 2-Chlor-5-methyl-pyridin, 3-Methyl-pyridin-1-oxid mit Phosphorsäuredialkylamiddichlorid oder Phosphorsäurealkylesterdichlorid unter Zugabe von Diisopropylamin als Säureakzeptor in Methylenchlorid oder Chlorbenzol umsetzt.

## Claims

1. Process for the preparation of substituted 2-chloropyridine derivatives of the formula (I) 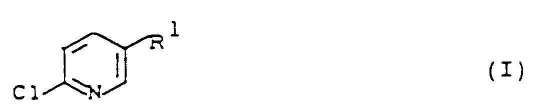 in which
R¹ represents chlorine, alkyl(C₁-C₄), halogenoalkyl(C₁-C₄), cyanoalkyl(C₁-C₄), alkoxy(C₁-C₄)alkyl(C₁-C₄), dialkylamino(C₁-C₄)alkyl(C₁-C₄), carbalkoxy(C₁-C₄) or 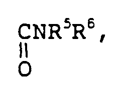 where R⁵ and R⁶ are identical or different and represent hydrogen or alkyl (C₁-C₄)
characterized in that pyridine 1-oxides of the formula (II) 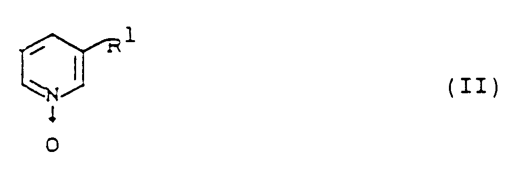 in which
R¹ has the abovementioned meaning,
are reacted with a chlorine-containing phosphoric acid derivative from the series of the chlorophosphoric esters and chlorophosphoramides in the presence of an inert organic solvent and in the presence of an acid acceptor at temperatures between -20°C and 200°C, and the resulting product is separated further, if appropriate.

2. Process according to Claim 1, characterized in that in the reaction
monoalkyl chlorophosphate,
dialkyl chlorophosphate,
cycloalkyl chlorophosphate,
aryl chlorophosphate,
diaryl chlorophosphate,
N-monoalkyl-chlorophosphoramide,
N,N-dialkyl-chlorophosphoramide,
N,N,N',N'-tetralkylchlorophosphoric diamide,
N-cycloalkyl-chlorophosphoramide,
N-aryl-chlorophosphoramides or
N,N-diaryl-chlorophosphoramide
is employed as the chlorine-containing phosphoric acid derivative.

3. Process according to Claims 1 and 2, characterized in that a basic organic nitrogen compound from the series comprising the dialkylamines, trialkylamines, dialkyl-cycloalkylamines, dialkylaralkylamines, dialkylarylamines and pyridine derivatives is employed as the acid acceptor.

4. Process according to Claims 1 to 3, characterized in that diisopropylamine is employed as the acid
acceptor.

5. Process according to Claims 1 to 4, characterized in that methylene chloride, chloroform or chlorobenzenes are employed as the inert organic solvents.

6. Process according to Claims 1 to 5, characterized in that the reaction is carried out at temperatures between 0°C and 150°C.

7. Process according to Claims 1 to 6, characterized in that an equimolar mixture of 1 to 10 moles of a chlorophosphoric ester or chlorophosphoramide and an amine in each case is added dropwise to a solution of 1 mole of the substituted pyridine-N-oxide of the formula (III) in a diluent, in a diluent, and the reaction mixture is stirred for several hours.

8. Process according to Claims 1 to 7, characterized in that the reaction mixture formed in the reaction is distilled, extracted or subjected to steam distillation.

9. Process according to Claims 1 to 8, characterized in that 3-methyl-pyridine-1-oxide is reacted with N,N-dialkyl-dichlorophosphoramide or alkyl dichlorophosphate in methylene chloride or chlorobenzene with the addition of diisopropylamine as the acid acceptor, for the preparation of 2-chloro-5-methyl-pyridine.

## Revendications

1. Procédé de fabrication de dérivés de la 2-chloropyridine substitués de la formule (I) 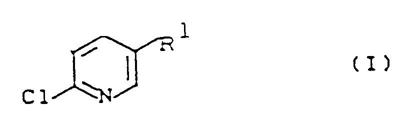 dans laquelle
R¹ représente le chlore, un alkyle (de C₁ à C₄), un halogénure d'alkyle (de C₁ à C₄), un cyanalkyle (de C₁ à C₄), un alcoxy (de C₁ à C₄) alkyle (de C₁ à C₄), un dialkylamino (de C₁ à C₄) alkyle (de C₁ à C₄), un carbalcoxy (de C₁ à C₄) ou 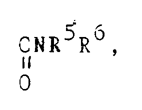 dans laquelle R⁵ et R⁶ sont identiques ou différents et représentent l'hydrogène ou un alkyle (de C₁ à C₄),
caractérisé en ce que l'on fait réagir des oxydes de pyridine-1 de la formule (II) 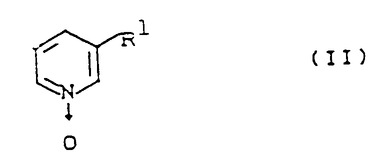 dans laquelle
R¹ possède la valeur indiquée ci-dessus,
avec un dérivé chloré de l'acide phosphorique de la série des chlorures d'esters de l'acide phosphorique et des chlorures d'amides de l'acides phosphorique, en présence d'un solvant organique inerte et d'un accepteur d'acide, à des températures comprises entre - 20 °C et 200 °C, et en ce que l'on soumet éventuellement le produit obtenu à une étape de séparation supplémentaire.

2. Procédé selon la revendication 1, caractérisé en ce que l'on met en oeuvre comme dérivé chloré de l'acide phosphorique dans la réaction, du
chlorure d'ester monoalkylique de l'acide phosphorique
chlorure d'ester dialkylique de l'acide phosphorique
chlorure d'ester cycloalkylique de l'acide phosphorique
chlorure d'ester arylique de l'acide phosphorique
chlorure d'ester diarylique de l'acide phosphorique
chlorure d'amide monoalkylique de l'acide phosphorique
chlorure d'amide dialkylique de l'acide phosphorique
chlorure de bis (amide dialkylique) de l'acide phosphorique
chlorure d'amide cycloalkylique de l'acide phosphorique
chlorure d'amide arylique de l'acide phosphorique
chlorure d'amide diarylique de l'acide phosphorique.

3. Procédé selon les revendications 1 et 2, caractérisé en ce que l'on met en oeuvre comme accepteurs d'acides, un composé azoté organique basique de la série des dialkylamines, trialkylamines, dialkyl-cycloalkylamines, dialkylaralkylamines, dialkylarylamines et des dérivés pyridiques.

4. Procédé selon les revendications 1 à 3, caractérisé en ce que l'on met en oeuvre comme accepteur d'acide, de la diisopropylamine.

5. Procédé selon les revendications 1 à 4, caractérisé en ce que l'on met en oeuvre comme solvants organiques inertes, du chlorure de méthylène, du chloroforme ou du chlorobenzène.

6. Procédé selon les revendications 1 à 5, caractérisé en ce que la réaction est exécutée à des températures comprises entre 0 et 150 °C.

7. Procédé selon les revendications 1 à 6, caractérisé en ce l'on ajoute goutte à goutte à une solution de 1 mole de l'oxyde de pyridine-1 substitué de la formule (II) dans un diluant, un mélange équimolaire de 1 à 10 moles dans chaque cas d'un chlorure d'ester de l'acide phosphorique ou d'un chlorure d'amide de l'acide phosphorique et d'une amine et en ce que l'on agite le mélange réactionnel pendant plusieurs heures.

8. Procédé selon les revendications 1 à 7, caractérisé en ce l'on soumet le mélange résultant de la réaction à une distillation, à une extraction ou à un entraînement à la vapeur d'eau.

9. Procédé selon les revendications 1 à 8, caractérisé en ce que pour fabriquer de la 2-chloro-5-méthyl-pyridine, on fait réagir de l'oxyde de 3-méthyl-pyridine-1 avec du dichlorure de dialkylamide de l'acide phosphorique ou du dichlorure d'ester alkylique de l'acide phosphorique dans du chlorure de méthylène ou du chlorobenzène, en ajoutant de la diisopropylamine comme accepteur d'acide.
